# EUROPEAN PATENT SPECIFICATION

(11) **EP 1 370 697 B1**
(45) Date of publication and mention of the grant of the patent: **29.10.2008**
(21) Application number: 02731136.4
(22) Date of filing: 18.03.2002
(51) Int. Cl.: A61K 38/44, A61P 31/12, A61P 31/18

(54) **PEROXIREDOXIN DRUGS FOR TREATMENT OF HIV-1 INFECTION**
PEROXIREDOXIN-ARZNEISTOFFE ZUR BEHANDLUNG EINER HIV-1-INFEKTION
MEDICAMENTS A BASE DE PEROXYREDOXINE POUR LE TRAITEMENT D'INFECTIONS PAR LE VIH-1

(30) Priority: 23.03.2001 US 278234 P; 25.01.2002 US 57593
(43) Date of publication of application: 17.12.2003
(73) Proprietor: THE GENERAL HOSPITAL CORPORATION, Boston, MA 02114 (US)
(72) Inventor: LYNN, Ralf, Geiben, Watertown, MA 02472 (US); WALKER, Bruce, D., Milton, MA 02186 (US)
(74) Representative: Hartz, Nikolai
(86) International application number: PCT/US2002/008077
(87) International publication number: WO 2002/077294

(56) References cited:
- WO-A-96/39424
- US-A- 5 610 286
- US-A- 6 150 135
- US-B1- 6 352 836
- GEIBEN-LYNN RALF ET AL: "HIV-1 antiviral activity of recombinant natural killer cell enhancing factors, NKEF-A and NKEF-B, members of the peroxiredoxin family." JOURNAL OF BIOLOGICAL CHEMISTRY, vol. 278, no. 3, 17 January 2003 (2003-01-17), pages 1569-1574, XP002269832 ISSN: 0021-9258
- JIN, DONG-YAN ET AL: "Peroxiredoxins in cell signaling and HIV infection" ANTIOXIDANT AND REDOX REGULATION OF GENES (2000), 381-407. EDITOR(S): SEN, CHANDAN K.;SIES, HELMUT; BAEUERLE, PATRICK A. PUBLISHER: ACADEMIC PRESS, SAN DIEGO, CALIF. , XP001179350
- JIN D-Y ET AL: "Regulatory role for a novel human thioredoxin peroxidase in NF-[kappa]B activation" JOURNAL OF BIOLOGICAL CHEMISTRY 1997 UNITED STATES, vol. 272, no. 49, 1997, pages 30952-30961, XP002269833 ISSN: 0021-9258

## Description

### FIELD OF THE INVENTION

The present invention relates to the use of specific peroxiredoxins.

### BACKGROUND OF THE INVENTION

The human retrovirus, human immunodeficiency virus (HIV) causes Acquired Immunodeficiency Syndrome (AIDS), an incurable disease in which the body's immune system breaks down leaving the victim vulnerable to opportunistic infections, *e*.*g*., pneumonia, and certain cancers, *e*.*g*., Karposi's Sarcoma. AIDS is major global health problem. The Joint United Nations Program on HIV/AIDS (UNAIDS) estimates that there are now over 34 million people living with HIV or AIDS worldwide, some 28.1 million of those infected individuals reside in impoverished sub-Saharan Africa. In the United States, one out of every 250 people are infected with HIV or have AIDS. Since the beginning of the epidemic, AIDS has killed nearly 19 million people worldwide, including some 425,000 Americans. AIDS has replaced malaria and tuberculosis as the world's deadliest infectious disease among adults and is the fourth leading cause of death worldwide.

There is still no cure for AIDS. There is, however, an armamentarium of antiretroviral drugs that prevent HIV from reproducing and ravaging the body's immune system. One such class of drugs are the reverse transcriptase inhibitors, *e*.*g*., abacavir, delaviridine, didanosine, efavirenz, lamivudine, nevirapine, stavudine, zalcitabine, and zidovudine, which attack an HIV enzyme called reverse transcriptase. Another class of drugs is the protease inhibitors, *e*.*g*., amprenavir, indinavir, nelfinavir, ritonavir, and saquinavir, which inhibit HIV enzyme protease. First introduced in 1995, these protease inhibitors are widely used for the treatment of HIV infection alone or in combination with other antiretroviral drugs. Today, approximately 215,000 of the estimated 350,000 patients receiving treatment for HIV infection in the United States take at least one protease inhibitor.

Highly active antiretroviral drug therapy (HEART) is a widely used anti-HIV therapy that entails triple-drug protease inhibitor-containing regimens that can completely suppress viral replication (Stephenson, JAMA, 277: 614-6 (1997)). The persistence of latent HIV in the body, however, has been underestimated. It is now recognized that there exists a reservoir of HIV in perhaps tens of thousands to a million long-lived resting "memory " T lymphocyte (CD4), in which the HIV genome is integrated into the cells own DNA (Stephenson, JAMA, 279: 641-2 (1998)). This pool of latently infected cells is likely established during primary infection.

Such combination therapy is often only partially effective, and it is unknown how much viral suppression is required to achieve durable virologic, immunologic, and clinical benefit (Deeks, JAMA, 286: 224-6 (2001)). Anti-HIV drugs are highly toxic and can cause serious side effects, including heart damage, kidney failure, and osteoporosis. Long-term use of protease inhibitors has been linked to peripheral wasting accompanied by abnormal deposits of body fat. Other manifestations of metabolic disruptions associated with protease inhibitors include increased levels of triglycerides and cholesterol, pancreatitis, atherosclerosis, and insulin resistance (Carr et al., LANCET, 351: 1881-3 (1998)). The efficacy of current anti-HIV therapy is further limited by the complexity of regimens, pill burden, and drug-drug interactions. Compliance with the toxic effects of antiretroviral drugs make a lifetime of combination therapy a difficult prospect and many patients cannot tolerate long-term treatment with HAART. There is an urgent need for other antiviral therapies due to poor adherence to combination therapy regimes, which has led to the emergence of drug-resistant strains of HIV. Other drugs may improve compliance by substantially reducing the daily "pill burden" and simplifying the complicated dietary guidelines associated with the use of current protease inhibitors.

The HIV virus enters the body of an infected individual and lives and replicates primarily in the white blood cells. The hallmark of HIV infection, therefore, is a decrease in cells called T-helper or CD4 cells of the immune system. The molecular mechanism of HIV entry into cells involves specific interactions between the viral envelope glycoproteins (env) and two target cell proteins, CD4 and a chemokine receptor. HIV cell tropism is determined by the specificity of the env for a particular chemokine receptor (Steinberger et al., PROC. NATAL. ACAD. SCI. USA. 97: 805-10 (2000)). T-cell-line-tropic (T-tropic) viruses (X4 viruses) require the chemokine receptor CXR4 for entry. Macrophage (M)-tropic viruses (R5 viruses) use CCR5 for entry (Berger et al., NATURE, 391: 240 (1998)). M-tropism is linked to various aspects of AIDS, including AIDS dementia, and may be important in disseminating the virus throughout the body and serving as a reservoir of virus in the body.

CD8⁺ T-cells inhibit HIV-1 replication by both cytolytic and non-cytolytic mechanisms (Yang et al., ADV IMMUNOL. 66: 273-311 (1997)). The importance of cell-mediated cytotoxic immunity for the partial control of human immunodeficiency virus type 1 (HIV-1) replication in infected individuals is now widely recognized (Harrer et al., AIDS RES HUM RETROVIRUSES 12: 585-592 (1996); Fowke et al., IMMUNOL CELL BIOL 78: 586-595 (2000); McMichael. CELL 93: 673-676 (1998)). The direct killing of virus-infected cells by antigen-specific, cytotoxic T-lymphocytes (CTL) is considered to be the dominant mechanism of virus suppression. Nevertheless, chemokines (*e*.*g*., MIP-1α, MIP-1β, RANGES, I-309) produced by CD8⁺ T-cells have been shown to inhibit R5- and X4-virus HIV-1 replication *in vitro* (Bleul et al., NATURE 382: 829-833 (1996); Cocchi et al., SCIENCE 270: 1811-1815 (1995); Horuk et al., J BIOL CHEM 273: 386-391 (1998); Pal et al., SCIENCE 278: 695-698 (1997)) at the level of viral entry (Garzino-Demo et al., PROC NATL ACAD SCI USA 96: 11986-11991 (1999); Samson et al., NATURE 382: 722-725 (1996)) and may play a critical role *in vivo* as an antiviral host defense (Furci et al., J EXP MED 186: 455-460 (1997). CD8⁺ T-lymphocytes can suppress human immunodeficiency virus type I (HIV-1) replication *in vitro* by secreting a soluble factor(s), which differs from the chemokines in the mechanism of inhibition. This factor, which may be useful in preventing or reversing HIV-1 infection and AIDS (acquired immune deficiency syndrome) progression, remains undefined (*e*.*g*., Walker et al., SCIENCE 234: 2563-2566 (1986); Tomaras et al., PROC NATL ACAD SCI USA 97: 3503-3508 (2000)) and has been termed CD8⁺ T-lymphocyte antiviral factor (CAF). The present lack of understanding regarding the identity of one or more cytokines or chemokines secreted by CD8⁺ T-cells is a serious deficiency in the field of HIV-1 infection and treatment.

Whereas the CTL response is major histocompatibility complex (MHC) class I restricted, this restriction does not apply to inhibition of HIV-1 replication by CAF (Walker et al., J VIROL 65: 5921-5927 (1991). Moreover, the production of CAF appears to be a property of stimulated CD8⁺ T-cells and does not require HIV infection (Geiben-Lynn et a/., J VIROL 75: 8306-8316 (2001); Castro et al., CELL IMMUNOL 132: 246-255 (1991)). One site of CAF action is the inhibition of HIV-1 RNA transcription, particularly at the step of long terminal repeat (LTR)-driven gene expression, which is assumed to function through down-regulation of the NF-κB pathway. It seems probable that the antiviral action of CAF is achieved by more than one cytokine or chemokine secreted by CD8⁺ T-cells, perhaps acting in concert (Moriuchi et al., PROC NATL ACAD SCIUSA 93: 15341-15345 (1996); Bailer et al., BUR J IMMUNOL 30: 1340-1349 (2000)).

It would therefore be useful to identify one or more factors secreted by CD8⁺ T-cells that suppress HIV-1 infectivity, in order to block the onset and/or progression of AIDS.

### SUMMARY OF THE INVENTION

The present invention provides a use according to claim 1.

The present invention provides new capacities and abilities in the reduction or elimination of HIV-1 infectivity as well as the prevention and/or treatment of acquired immune deficiency syndrome (AIDS) in a mammal, such as a human subject in need thereof.

The present disclosure provides in part a method of treating HIV -1 infection. In one embodiment, the method comprises contacting a cell susceptible to HIV-1 infection with a peroxiredoxin or a peptide fragment in am amount sufficient to inhibit infection of the cell by the HIV-1.

The present disclosure also provides a method of inhibiting or decreasing HIV-1 infectivity using a substantially purified preparation of a peroxiredoxin in an amount sufficient to decrease the infectivity of HIV-1 in the biological sample. In a preferred embodiment, biological samples are contacted with at least about 5 µg/ml final biological sample volume preparations of peroxiredoxin. Biological samples which may treated for HIV-1 infection include, but are not limited to, blood, plasma, serum, saliva, semen, cervical secretions, saliva, urine, breast milk, and amniotic fluids.

The present disclosure also provides kits, such as a kit comprising, in one or more containers, a peroxiredoxin-based pharmaceutical composition, such as for the treatment and/or prevention of AIDS.

These and other objects of the present invention will be apparent from the detailed description of the invention provided below.

### BRIEF DESCRIPTION OF THE DRAWINGS

The present invention will be further understood from the following description with reference to the figures in which:

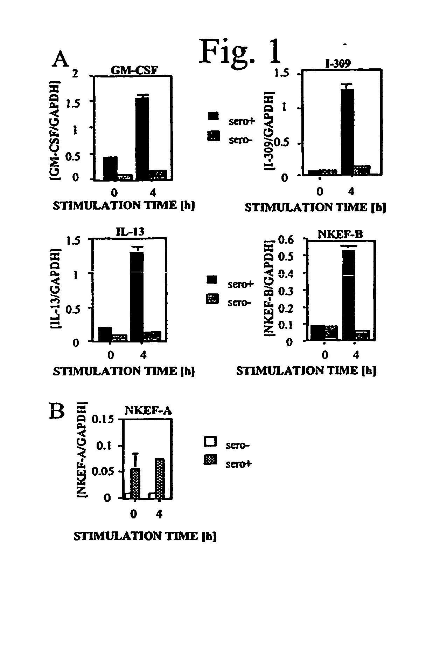

FIG 1 demonstrates the differential gene expression (p<0.001) between CD3 crosslinked CD8⁺ T-cells of seronegative individuals and HIV-1 untreated patients using the Atlas Array^{™} (A) and Northern-blot analysis of the NKEF-A transcription (B). (A) The Atlas Array^{™} (Clontech) used is a nitrocellulose membrane with 588 spotted cDNAs (see Materials and Methods). Only the four genes shown with cytokines GM-CSF, IL-13, chemokine I-309 (CLL1) and peroxiredoxin NKEF-B showed significantly different gene expression (p<0.001). (B) Northern blot analysis of NKEF-A gene expression shows a significantly higher expression after 4 h anti-CD3 stimulation in CD8⁺ T-cells of HIV-1 infected but untreated patients compared to CD8⁺ T-cells of seronegative individuals.

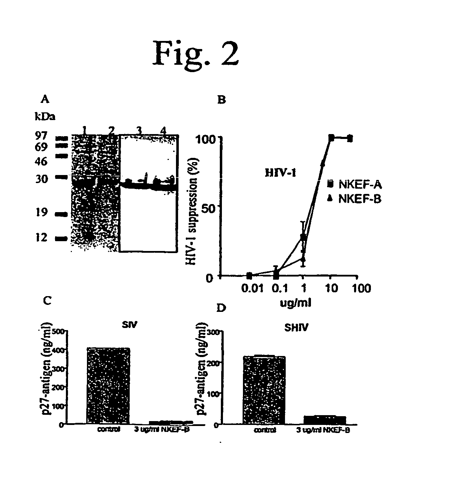

FIG 2 shows an SDS-PAGE of purified NKEF-A and NKEF-B (A), NKEF-A and NKEF-B protein inhibition ofT cell tropic HIV-1 replication (B), and inhibition of SIV and SHIV strains (C, D). (A) Coomassie stained SDS-PAGE with NKEF-A (lane 1) and NKEF-B (lane 2) and Western-blot of NKEF-A (lane 3) and NKEF-B (lane 4) using the polyclonal NKEF-A/NKEF-B antibody. (B) Dose response curve of NKEF-A and NKEF-B. At a dose of 1 µg/ml, NKEF-A appeared to inhibit HIV-1 replication in H9 CD4⁺ T-cells to a greater degree than NKEF-B. However, at 10 µg/ml or above, both NKEF-A and NKEF-B nearly eliminated HIV-1 infectivity (>95% inhibition). Since it is hypothesized that the thiol redox reactions catalyzed by Prx is responsible for the antiviral effect, it is expected that other members of the Prx family are also candidate antiviral compounds. The results illustrate the antiviral effects of peroxiredoxins against HIV. (C, D) p27 protein measurements of SIV and SHIV after NKEF-B treatment in three independent experiments after 9 day.

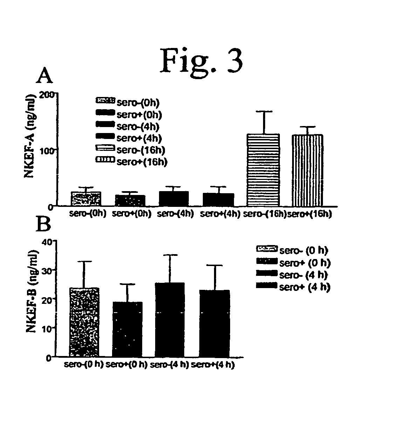

FIG 3 is a secretion profile of NKEF-A (A) and NKEF-B (B) by CD8⁺ T-cells of seropositive individuals. NKEF-A and NKEF-B protein amount was measured by a specific BLISA without (0 h), with 4 h or 16 h anti-CD3 stimulation. Time zero denotes 4 h supernatant without anti-CD3 stimulation.

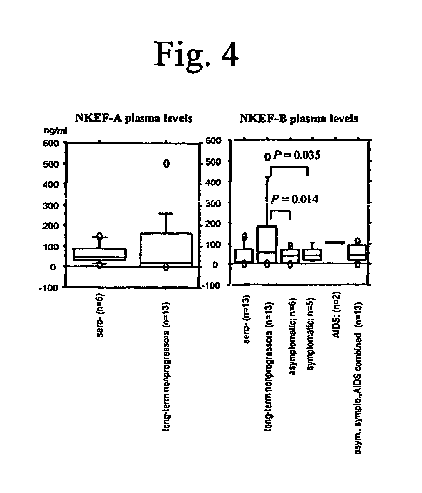

FIG 4 details box plots showing the 10^{th}, 25^{th}, 50^{th} (median), 75^{th}, and 90^{th} percentiles, of NKEF-A (A) and NKEF-B (B) plasma concentrations of seronegative individuals, HIV infected but untreated, asymptomatic (Phase A), symptomatic (Phase B) and AIDS (Phase C) patients.

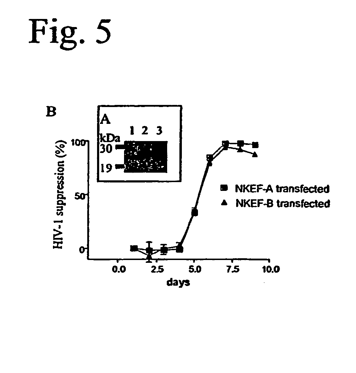

FIG 5 indicates NKEF-A and NKEF-B protein production of transfected T-cells (A) and T-cell protection through NKEF-A and NKEF-B gene transfection (B). (A) NKEF-A and NKEF-B protein expression of 2x10⁵ untransfected (lane 1), NKEF-A (lane 2) and NKEF-B transfected (lane 3) Jurkat CD4⁺ cells in a Western-blot with the polyclonal NKEF-A/NKEF-B antibodies. Binding of antibodies was visualized on an autoradiography film (see materials and methods). (B) NKEF-A and NKEF-B transfected Jurkat CD4⁺ cells showed protection against HIV-1 infection in the inhibition test used starting at day 5 after transfection. T-cells are strongly protected (80 - 98%) at day 6-9 after transfection.

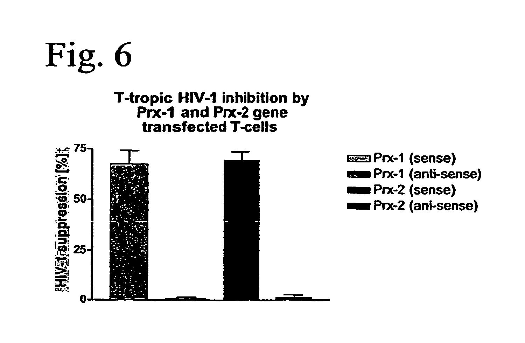

FIG 6 indicates NKEF-A (Prx-I) and NKEF-B (Prx-II) and T-cell protection through NKEF-A and NKEF-B gene transfection. NKEF-A and NKEF-B transfected H9 CD4⁺ cells showed protection against HIV-1 infection in the inhibition test used compared with cells transfected with NKEF-A and NKEF-B anti-sense constructs. T-cells are strongly protected (70%) at day 6 after transfection.

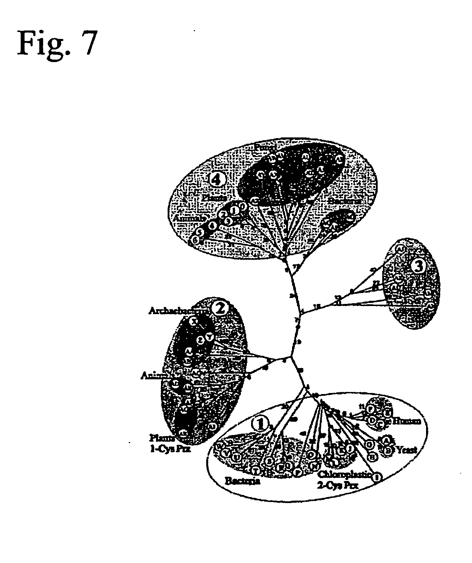

FIG. 7 shows the phylogenetic tree of type I and type II peroxiredoxins. See Verdoucq et al., JBC, 274: 19714-19722 (1999). The DARWIN software was used to generate this tree. The GenBank or EMBL accession number is indicated for each represented organism. Cluster 1: 2-Cys thiol peroxidases. Yeast: A, P34760 YML028 S. cerevisiae; B, 927720 YDR453 S. cerevisiae. Human: C, L19185 HKEFA Homo sapiens; D, Q06830 PAG H. sapiens; E, U25182 AOE37 H. sapiens; F, X82321 TSAOX H. sapiens; G, U26666 Trypanosoma brucei; H, U88577 Fasciola hepatica; I, S67947 Entamoeba histolytica. Chloroplatic 2-Cys Prx: J, Q96291 A. thaliana; K, Z34917 BAS1 Hordeum; L, D64000 orf s11010785 BAS1 Synechocystis; M, U38804 BAS1 Porphyra. Bacteria: N, M60116 Salmonella typhimurium; O, Z99111 Bacillus subtilis; P, AE000654 Helicobacter pylori; Q, U24084 Mycobacterium bovis; R, U31978 Mycobacterium smegmatis; S, U18620 Corynebacterium diphtheria; T, U94336 Xanthomonas campestris; U, A35441 Salmonella typhimurium; V, S52934 Staphylococcus aureus; W, P80239 B. subtilis. Cluster 2:, 1-Cys thiol peroxidases. Archaebacteria: X, AE000804 Methanobacterium fulgidus; Y, AE001087 Archaeolus fulgidus; Z, H64391 Methanococcus jannaschii; AA, U36479 Sulfolobus; AB, AF007757 Sulfolobus metallicus; AC, P34227 YLB064 Saccharomyces cerevisiae. Animals: AD, P30041 H. sapiens; AE, P52570 Rehydrin Onchocerca. Plants: AF, P52571 Rehydrin Bromus; AG, Y12089 Rehydrin A. thaliana; AH, U40818 Rehydrin Tortula ruralis; AI, D1018112 Synechocystis. Cluster 3, bacterioferritin comigrating proteins. AJ, AL021185 Mycobacterium; AK, U32711 Hemophilus; AL, M63654 E. coli; AM, U14189 Plasmodium falci; AN, P40553 YIL010 S. cerevisiae. Cluster 4, YLR109 and homologues. Bacteria, AO, U32739 Heamophilus; AP, D90909 Synechocystis; AQ, X72888 orf sll1621 Rhizobium capsulatus. Fungi, AR, AJ002536 Schizosaccharomyces; AS, U53878 YLR109 S. cerevisiae; AT, U11244 Lipomyces; AU, AB011805 Malassezia furfur; AV, P14293 PMP20b C. boidini; AW, P14292 PMP20a C. boidini; AX, AB011804 M. furfur; AY, U58050 Aspergillus; AZ, EST contig translation Dictyostelium. Plants: 0, EST contig translation Rice; 1, EST contig translation Populus; 2, AtTPX2 A. thaliana; 3, AtTPX1 A. thaliana. Animals: 4, EST contig translation Drosophila; 5, EST contig translation H. sapiens; 6, EST contig translation mouse.

### DETAILED DESCRIPTION OF THE INVENTION

In the present invention, specific peroxiredoxins (Prx) as further defined by claim 1 have been found to be useful in causing a reduction or elimination in HIV-1 infectivity, and providing therapeutic benefit to patients at risk of or suffering from AIDS.

Peroxiredoxins (Prx) are antioxidant enzymes containing highly conserved cysteine residues, and are classified into two groups: first, those Prx members having a single cysteine at about position 47, and second, those Prx members having cysteines at about positions 47 and 170. There are six Prx subfamilies (Butterfield et al., ANTIOXID REDOX SIGNAL 1: 385-402, 1999), some members are also known as natural killer enhancing factors (NKEF-A (PrxI) and NKEF-B (PrxII)) because these proteins enhance cytocidal activity or natural killer cells (Shau et al., BBRC 199: 83-88, 1994; Rhee et al., BIOFACTORS 10: 207-209,1999). Peroxiredoxins are useful to counterbalance the toxic effects of reactive oxygen species (ROS). ROS, including O₂ radicals, H₂O₂ and OH radicals, are naturally generated from the incomplete reduction of oxygen during respiration, or from external sources such as light, ionizing radiation or certain drugs. Exposure of cells to such chemical or physical insults induces a "stress" response resulting in synthesis of heat shock proteins and many other protein including chaperons (proteins involved in protein folding and translocation) and antioxidant proteins (peroxiredoxins; *see*, *e*.*g*., Butterfield et al., ANTIOXID REDOX SIGNAL 1: 385-402, 1999). Unchecked ROS can lead to oxidative damage in the cell, including lipid peroxidation, protein modification, DNA base modification and DNA stand breaks. ROS can have also signal transduction functions involving regulation of cell functions like growth and differentiation, immune response or phagocytic cells and apoptosis. *See*, Finke1, CURR OPIN. CELL BIOL. 10: 248-253 (1998); Finkel, LEUKOC BIOL 65: 337-340 (1999); Karin et al., CANCER J. SCI AM 4 SUPPL 1: 92-99 (1998); Sen et al., FASAB J 10: 709-720 (1996). For example, the tumor suppressor protein p53, which helps maintain genomic stability by halting cell cycle progression under cellular stress conditions, is regulated by thiol oxidation state and thioredoxin reductase via Ref-1. *See*, Jayaraman et al., GENES DEV 11: 558-570 (1997). Another example is the signaling by ROS of the transcription factors Activator Protein-1 (AP-1) and Nuclear Factor κB (NF-κB). *See*, Hirota et al., PROC NATAL ACAD SCI USA 94: 3633-3638 (1997); Sen et al., FASEB J 10: 709-720 (1996). Also, imbalance between ROS and antioxidant defense mechanisms have been implicated in pathological situations like atherosclerosis, Alzheimer's disease, and HIV activation. *See,* Diaz et al., N ENGL J MED 337: 408-416 (1997); Ross, NATURE 362: 801-809 (1993).

Accordingly, the present invention provides uses of specific peroxiredoxins and allows methods to decrease or eliminate HIV-1 infectivity.

### Definitions

As used herein, each of the following terms has the meaning associated with it in this section.

As used herein, the term "substantially pure" describes a compound, *e*.*g*., a protein or polypeptide that has been separated from components which naturally accompany it. Typically, a compound is substantially pure when at least 10%, more preferably at least 20%, more preferably at least 50%, more preferably at least 60%, more preferably at least 75%, more preferably at least 90%, and most preferably at least 99% of the total material (by volume, by wet or dry weight, or by mole percent or mole fraction) in a sample is the compound of interest. Purity can be measured by any appropriate method, *e*.*g*., in the case of polypeptides by column chromatography, gel electrophoresis or HPLC analysis. A compound, e.g., a protein, is also substantially purified when it is essentially free of naturally associated components or when it is separated from the native contaminants which accompany it in its natural state. Included within the meaning of the term "substantially pure" as used herein is a compound, such as a protein or polypeptide, which is homogeneously pure, for example, where at least 95% of the total protein (by volume, by wet or dry weight, or by mole percent or mole fraction) in a sample is the protein or polypeptide of interest.

As used herein, the term "specific binding" or "specifically binds" means a protein, such as an antibody which recognizes and binds an peroxiredoxin or a ligand thereof, but does not substantially recognize or bind other molecules in a sample.

As used herein, the term "pharmaceutically acceptable carrier" means a chemical composition with which the active ingredient may be combined and which, following the combination, can be used to administer the active ingredient to a subject.

As used herein, the term "physiologically acceptable" ester or salt means an ester or salt form of the active ingredient which is compatible with any other ingredients of the pharmaceutical composition, which is not deleterious to the subject to which the composition is to be administered.

As used herein, an "oily" liquid is one which comprises a carbon-containing liquid molecule and which exhibits a less polar character than water.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed., REMINGTON'S PHARMACEUTICAL SCIENCES, Mack Publishing Co., Easton, Pa. (1985), which is incorporated herein by reference.

The term "transformation" means introducing DNA into a suitable host cell so that the DNA is replicable, either as an extrachromosomal element, or by chromosomal integration. The term "transfection" refers to the taking up of an expression vector by a suitable host cell, whether or not any coding sequences are in fact expressed.

The term "infection" refers to the introduction of nucleic acids into a suitable host cell by use of a virus or viral vector.

The term "peroxiredoxin" refers to any member of the peroxiredoxin family and encompasses naturally occurring peroxiredoxins, as well as synthetic or recombinant peroxiredoxins. Further, the term "peroxiredoxin" encompasses allelic variants, species variants, and conservative amino acid substitution variants. The term also encompasses full-length peroxiredoxins, as well as peroxiredoxin fragments. It will thus be understood that fragments of peroxiredoxins variants, in amounts giving equivalent biological activity to full-length peroxiredoxins, can be used in the methods of the invention, if desired. Fragments of peroxiredoxin incorporate at least the amino acid residues of peroxiredoxins necessary for a biological activity similar to that of intact peroxiredoxin.

The term "peroxiredoxin" also encompasses variants and functional analogs of peroxiredoxins having a homologous amino acid sequence with a peroxiredoxin. The present invention thus includes pharmaceutical formulations comprising such peroxiredoxin variants and functional analogs, carrying modifications like substitutions, deletions, insertions, inversions or cyclisations, but nevertheless having substantially the biological activities of peroxiredoxins. The terms "fragment" and "analog" are used interchangeably herein to describe peroxiredoxins useful in the methods of the present invention.

According to the present invention, "homologous amino acid sequence" means an amino acid sequence that differs by one or more conservative amino acid substitutions, or by one or more non-conservative amino acid substitutions, deletions, or additions located at positions at which they do not destroy the biological activities of the polypeptide. Conservative amino acid substitutions typically include substitutions among amino acids of the same class. These classes include, for example, (a) amino acids having uncharged polar side chains, such as asparagine, glutamine, serine, threonine, and tyrosine; (b) amino acids having basic side chains, such as lysine, arginine, and histidine; (c) amino acids having acidic side chains, such as aspartic acid and glutamic acid; and (d) amino acids having nonpolar side chains, such as glycine, alanine, valine, leucine, isoleucine, proline, phenylalanine, methionine, tryptophan, and cysteine.

The term "peroxiredoxin" encompasses variants and functional analogs of peroxiredoxins having one or more conserved cysteine residues (*e*.*g*., at about position 47 (type I-peroxiredoxin) and/or 170 (type II peroxiredoxin). A peroxiredoxin includes known peroxiredoxin subfamilies (Prx I-VI) as well as yet-uncharacterized peroxiredoxins. A peroxiredoxin can be from a mammal (*e*.*g*., a human) or non-mammalian source (*e*.*g*., a bacteria). Peroxiredoxins include *e*.*g*., GenBank Accession Numbers AOE372, U25182, and L19185. Further, peroxiredoxins include proteins identified by other names, including thiol-specific antioxidant (TSA; Accession No. Z22548), proliferation-associated protein (Pag; Accession No. X67951), MER5 protein (MER5; Accession No. D49396), human MER5 protein (MER5-human; AccessionNo. D49396), mouse MER5 protein (MER5-mouse; Accession No. M28723), bovine antioxidant protein and substrate protein for mitochondrial ATP-dependent protease SP22 (SP22-bovine; Accession No. D82025), rat heme-binding 23-kDa protein (HBP23-rat; Accession No. D30035), human proliferation-associated gene product (pag-human; Accession No. X67951), mouse MSP-23 protein (MSP23-mouse; Accession No. D 16142), NKEF-A (Accession No. L19184), rat thioredoxin peroxidase (Tprx-rat; Accession No. U06099), mouse thioredoxin peroxidase (Tpx-mouse; Accession No. U20611), human ORF6 protein (ORF6-human; Accession No. D 14662), murine peroxiredoxin (Accession Nos.AF208729 and AF208730), Prx4 mouse homolog (Accession No. U96746) (See FIG. 7).

The specific peroxiredoxin used in the present invention need not be a naturally-occurring peroxiredoxin, and may be a variant peroxiredoxin. For example, the peroxiredoxin can contain any number (*e*.*g*., up to about 30) conservative amino acid substitutions, so long as the anti-HIV activity of peroxiredoxin is retained. A conservative amino acid substitution is one in which an amino acid is replaced with another residue having a chemically similar side chain. Families of amino acids having similar side chains have been defined in the art. Conservative amino acid substitutions typically include substitutions among amino acids of the same class. These classes include, for example, (a) amino acids having uncharged polar side chains, such as asparagine, glutamine, serine, threonine, and tyrosine; (b) amino acids having basic side chains, such as lysine, arginine, and histidine; (c) amino acids having acidic side chains, such as aspartic acid and glutamic acid; and (d) amino acids having nonpolar side chains, such as glycine, alanine, valine, leucine, isoleucine, proline, phenylalanine, methionine, tryptophan, and cysteine.

It is possible that the amino acid sequences around the cysteine at about position 47 of the peroxiredoxin may contribute to the anti-HIV activity; thus in some embodiments this region of redox activity are retained in the peroxiredoxin. In other embodiments, only the specific domain of the peroxiredoxin conferring anti-HIV activity of the peroxiredoxin is retained; this anti-HIV domain may be contiguous or separate from the peroxiredoxin domain conferring the redox activity.

The peroxiredoxin is at least 90% (or 95%) identical with the amino acid sequence of a naturally occurring specific peroxiredoxin. Identity is determined using the algorithm of Karlin and Altschul (PROC NATL ACAD SCI USA 87: 2264-2268 (1990)).

The invention includes the use of a composition comprising substantially purified peroxiredoxin as defined in claim 1. Peroxiredoxin is capable of inhibiting the infectivity of HIV-1 as described herein, and thus is useful in methods for the prevention of HIV-1 infection in a patient or for inhibiting the infectivity of HIV-1 containing bodily fluids. The peroxiredoxin to be used in the present invention is not particularly limited as long as it has been purified to the extent that it can be used as a pharmaceutical agent. For example, it can be purified from whole blood, blood plasma, serum or serum obtained by compression of coagulated blood. The starting material for preparing peroxiredoxin may be, for example, fraction IV-1 or IV, or supernatant I or II+III obtained by Cohn's fractionation of blood plasma. A commercially available peroxiredoxin preparation can be used.

Compositions comprising substantially purified peroxiredoxin may include peroxiredoxin alone, or in combination with other proteins. Peroxiredoxin may be substantially purified by any of the methods well known to those skilled in the art. Substantially pure protein may be purified by following known procedures for protein purification, wherein an immunological, chromatographic, enzymatic or other assay is used to monitor purification at each stage in the procedure. Protein purification methods are well known in the art, and are described, for example in Deutscher et al., (ed., GUIDE TO PROTEIN PURIFICATION, Harcourt Brace Jovanovich, San Diego (1990)). Peroxiredoxin can be purified by a method described in, for example, U.S. Pat No. 5,250,295. See also Sauri et al., J LEUKOC BIOL 59: 925-31, (1996).

The peroxiredoxin of the invention is, in one embodiment, used as a component of a pharmaceutical composition, which may also comprise buffers, salts, other proteins, and other ingredients acceptable as a pharmaceutical composition. The invention also includes the use of a modified form of peroxiredoxin, which is capable of contacting HIV-1 and inhibiting the infectivity of HIV-1 as described herein. The modified peroxiredoxin may be used as a component of a composition for use in a method for prevention of HIV-1 infection of a patient or in the inhibition of HIV-1 infectivity of biological fluids. A modified peroxiredoxin may include one or more amino acid additions, substitutions, deletions or modifications (*e*.*g*., alterations in glycosylation or phosphoryation). The N-terminus of the protein region (FFYLDFTFVCPTEI; SEQ ID NO: 1) and the C-terminus region (HGEVCPA; SEQ ID NO: 2) are conserved in the subfamilies and may be associated with the redox activity of peroxiredoxin (Butterfield et al., ANTIOXID REDOX SIGNAL 1: 385-402, (1999)). Modified peroxiredoxins used in the present invention include any polypeptide containing SEQ ID NO: 1 or SEQ ID NO: 2. Additionally, when thioredoxin is cleaved by macrophages to the inflammatory cytokine, eosinophil-cytotoxicity-enhancing factor, HIV replication is increased (Newman et al., J EXPERIMENT MED 180: 359-363 (1994)). A modified peroxiredoxin that is resistant to enzymatic cleavage (*e.g.,* by macrophages) is also included within the present invention. A peroxiredoxin of the invention includes a fusion protein containing the redox activity region(s) of peroxiredoxin.

The specific peroxiredoxin of the present invention can be used as an antiviral drug against other viruses (*e*.*g*., HTL-1, -2, HSV, EBV, HBV, HCV, or CMV), or other viruses involving transcriptional machinery similar to that of HIV-1, *See,* Sodroski et al., SCIENCE 225: 381-385, (1984).

The peroxiredoxin used in the invention may be a molecule, which comprises the protein or its peptide fragments alone, or may include other components, such as protein or other carbohydrate, or another molecule, which may be covalently linked to the peroxiredoxin, or may be non-covalently associated with the peroxiredoxin.

In another embodiment, the peroxiredoxin used in the invention may be prepared using a biochemical synthesis method. Biochemical methods for synthesizing proteins are well known to those skilled in the art.

The ability to contact HIV-1 virion may be assessed using assays described herein in the Examples section. For example, the virus may be incubated with the molecule comprising an peroxiredoxin of the invention, placed over a sucrose cushion and centrifuged. The virus pellet obtained is resuspended, concentrated with trichloroacetic acid (TCA) to concentrate the proteins, and aliquots of the pellet and supernatant are analyzed by Western blotting using antibodies to p24 (Nagashurmugam and Friedman, DNA CELL BIOL. 15: 353-361 (1996) or by an ELISA method.

In one embodiment, the molecule comprising the specific peroxiredoxin used according to the invention is capable of inhibiting the infectivity of HIV-1 in a patient by contacting an HIV-1 virion. The molecule comprising the peroxiredoxin is included as a component in a pharmaceutical composition, which may be administered to a patient to inhibit HIV-1 infectivity or to prevent infection by HIV-1. The inhibition of infectivity of HIV-1 by the molecule comprising the peroxiredoxin of the invention may be assessed as described herein. Such methods may include p24 assay, reverse transcriptase activity assay or TCID₅₀.

The disclosure also includes an antibody that is capable of specifically binding to peroxiredoxin. The antibody may be a monoclonal or a polyclonal antibody, or may be a synthetic, humanized or phage displayed antibody. The term "antibody," as used herein, refers to an immunoglobulin molecule that is able to specifically bind to a specific epitope on an antigen. Antibodies can be intact immunoglobulins derived from natural sources or from recombinant sources and can be immunoreactive portions of intact immunoglobulins. Antibodies are typically tetramers of immunoglobulin molecules. The antibodies may exist in a variety of forms including, for example, polyclonal antibodies, monoclonal antibodies, Fv, Fab and F(ab)₂, as well as single chain antibodies and humanized antibodies (Harlow et al., ANTIBODIES: A LABORATORY MANUAL, Cold Spring Harbor, N.Y. (1988); Houston et al., PROC. NATL. ACAD. ScI. USA 85: 5879-5883 (1988); Bird et al., SCIENCE, 242: 423-426 (1988)). By the term "synthetic antibody" as used herein, is meant an antibody which is generated using recombinant DNA technology, such as, for example, an antibody expressed by a bacteriophage as described herein. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using synthetic DNA or amino acid sequence technology which is available and well known in the art.

The disclosure also includes a method of inhibiting the infectivity of HIV-1 in bodily fluids, or in infective oral secretions. The method is useful in preventing HIV-1 infection, or inhibiting the infectivity of HIV-1. This method can be used, for example to inhibit the infectivity of biological fluids, for example in a hospital setting where medical personnel are exposed to infectious HIV-1 secretions.

In one embodiment, the method comprises contacting an HIV-1 virion with the human or non-human peroxiredoxin compositions described herein. In one embodiment, the peroxiredoxin composition may comprise substantially purified peroxiredoxin. The sample from a patient containing the HIV-1 virion may be obtained from any sample of bodily fluid, such as blood, plasma, serum, saliva, semen, cervical secretions, saliva, urine, breast milk, or amniotic fluids. In one embodiment, a composition comprising substantially purified peroxiredoxin is contacted with an HIV-1 virion from a sample of a patient for a period of time sufficient for the peroxiredoxin to inhibit the infectivity of HIV-1. The inhibition of the infectivity of HIV-1 can be assessed as described herein in the Examples.

The invention encompasses the preparation and use of pharmaceutical compositions comprising a compound useful for the prevention of HIV infection or inhibition of HIV infectivity as an active ingredient. Such a pharmaceutical composition may consist of the active ingredient alone, in a form suitable for administration to a subject, or the pharmaceutical composition may comprise the active ingredient and one or more pharmaceutically acceptable carriers, one or more additional ingredients, or some combination of these. The active ingredient may be present in the pharmaceutical composition in the form of a physiologically acceptable ester or salt, such as in combination with a physiologically acceptable cation or anion, as is well known in the art. Further, the peroxiredoxin used in the present invention may contain pharmacologically acceptable additives (*e.g.,* carrier, excipient and diluent), stabilizers or components necessary for formulating preparations, which are generally used for pharmaceutical products, as long as it does not adversely affect the object of the present invention.

Examples of the additives and stabilizers include saccharides such as monosaccharides (*e*.*g*., glucose and fructose), disaccharides (*e*.*g*., sucrose, lactose and maltose) and sugar alcohols (*e*.*g*., mannitol and sorbitol); organic acids such as citric acid, malic acid and tartaric acid and salts thereof (*e*.*g*., sodium salt, potassium salt and calcium salt); amino acids such as glycine, aspartic acid and glutamic acid and salts thereof (*e.g.,* sodium salt); surfactants such as polyethylene glycol, polyoxyethylene-polyoxypropylene copolymer and polyoxyethylenesorbitan fatty acid ester, heparin; and albumin.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions that are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates.

Pharmaceutical compositions prepared in the invention may be prepared, packaged, or sold in formulations suitable for oral, rectal, vaginal, parenteral, topical, pulmonary, intranasal, buccal, ophthalmic, or another route of administration. The preferred mode is intravenous administration.

The peroxiredoxin and the above-mentioned ingredients are admixed as appropriate to give powder, granule, tablet, capsule, syrup, injection and the like. Other contemplated formulations include projected nanoparticles, liposomal preparations, resealed erythrocytes containing the active ingredient, and immunologically-based formulations.

A pharmaceutical composition of the invention may be prepared, packaged, or sold in bulk, as a single unit dose, or as a plurality of single unit doses. As used herein, a "unit dose" is discrete amount of the pharmaceutical composition comprising a predetermined amount of the active ingredient The amount of the active ingredient is generally equal to the dosage of the active ingredient, which would be administered to a subject, or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient

In addition to the active ingredient, a pharmaceutical composition of the invention may further comprise one or more additional pharmaceutically active agents.

Particularly contemplated additional agents include anti-emetics and scavengers such as cyanide and cyanate scavengers. Controlled- or sustained-release formulations of a pharmaceutical composition of the invention may be made using conventional technology.

A formulation of a pharmaceutical composition of the invention suitable for oral administration may be prepared, packaged, or sold in the form of a discrete solid dose unit including, but not limited to, a tablet, a hard or soft capsule, a cachet, a troche, or a lozenge, each containing a predetermined amount of the active ingredient Other formulations suitable for oral administration include, but are not limited to, a powdered or granular formulation, an aqueous or oily suspension, an aqueous or oily solution, or an emulsion.

A tablet comprising the active ingredient may, for example, be made by compressing or molding the active ingredient, optionally with one or more additional ingredients. Compressed tablets may be prepared by compressing, in a suitable device, the active ingredient in a free-ffowing form such as a powder or granular preparation, optionally mixed with one or more of a binder, a lubricant, an excipient, a surface active agent, and a dispersing agent. Molded tablets may be made by molding, in a suitable device, a mixture of the active ingredient, a pharmaceutically acceptable carrier, and at least sufficient liquid to moisten the mixture. Pharmaceutically acceptable excipients used in the manufacture of tablets include, but are not limited to, inert diluents, granulating and disintegrating agents, binding agents, and lubricating agents. Known dispersing agents include, but are not limited to, potato starch and sodium starch glycollate. Known surface active agents include, but are not limited to, sodium lauryl sulphate. Known diluents include, but are not limited to, calcium carbonate, sodium carbonate, lactose, microcrystalline cellulose, calcium phosphate, calcium hydrogen phosphate, and sodium phosphate. Known granulating and disintegrating agents include, but are not limited to, corn starch and alginic acid. Known binding agents include, but are not limited to, gelatin, acacia, pre-gelatinized maize starch, polyvinylpyrrolidone, and hydroxypropyl methylcellulose. Known lubricating agents include, but are not limited to, magnesium stearate, stearic acid, silica, and talc.

Tablets may be non-coated or they may be coated using known methods to achieve delayed disintegration in the gastrointestinal tract of a subject, thereby providing sustained release and absorption of the active ingredient. By way of example, a material such as glyceryl monostearate or glyceryl distearate may be used to coat tablets. Further by way of example, tablets may be coated using methods described in U.S. Pat. Nos. 4,256,108; 4,160,452; and 4,265,874 to form osmotically-controlled release tablets. Tablets may further comprise a sweetening agent, a flavoring agent, a coloring agent, a preservative, or some combination of these in order to provide pharmaceutically elegant and palatable preparation.

Hard capsules comprising the active ingredient may be made using a physiologically degradable composition, such as gelatin. Such hard capsules comprise the active ingredient, and may further comprise additional ingredients including, for example, an inert solid diluent such as calcium carbonate, calcium phosphate, or kaolin.

Soft gelatin capsules comprising the active ingredient may be made using a physiologically degradable composition, such as gelatin. Such soft capsules comprise the active ingredient, which may be mixed with water or an oil medium such as peanut oil, liquid paraffin, or olive oil.

Liquid formulations of a pharmaceutical composition of the invention which are suitable for oral administration may be prepared, packaged, and sold either in liquid form or in the form of a dry product intended for reconstitution with water or another suitable vehicle prior to use.

Liquid suspensions may be prepared using conventional methods to achieve suspension of the active ingredient in an aqueous or oily vehicle. Aqueous vehicles include, for example, water and isotonic saline. Oily vehicles include, for example, almond oil, oily esters, ethyl alcohol, vegetable oils such as arachis, olive, sesame, or coconut oil, fractionated vegetable oils, and mineral oils such as liquid paraffin. Liquid suspensions may further comprise one or more additional ingredients including, but not limited to, suspending agents, dispersing or wetting agents, emulsifying agents, demulcents, preservatives, buffers, salts, flavorings, coloring agents, and sweetening agents. Oily suspensions may further comprise a thickening agent. Known suspending agents include, but are not limited to, sorbitol syrup, hydrogenated edible fats, sodium alginate, polyvinylpyrrolidone, gum tragacanth, gum acacia, and cellulose derivatives such as sodium carboxymethylcellulose, methylcellulose, hydroxypropylmethylcellulose. Known dispersing or wetting agents include, but are not limited to, naturally-occurring phosphatides such as lecithin, condensation products of an alkylene oxide with a fatty acid, with a long chain aliphatic alcohol, with a partial ester derived from a fatty acid and a hexitol, or with a partial ester derived from a fatty acid and a hexitol anhydride (*e.g*., polyoxyethylene stearate, heptadecaethyleneoxycetanol, polyoxyethylene sorbitol monooleate, and polyoxyethylene sorbitan monooleate, respectively). Known emulsifying agents include, but are not limited to, lecithin and acacia. Known preservatives include, but are not limited to, methyl, ethyl, or n-propyl-para-hydroxybenzoates, ascorbic acid, and sorbic acid. Known sweetening agents include, for example, glycerol, propylene glycol, sorbitol, sucrose, and saccharin. Known thickening agents for oily suspensions include, for example, beeswax, hard paraffin, and cetyl alcohol.

Liquid solutions of the active ingredient in aqueous or oily solvents may be prepared in substantially the same manner as liquid suspensions, the primary difference being that the active ingredient is dissolved, rather than suspended in the solvent. Liquid solutions of the pharmaceutical composition may comprise each of the components described with regard to liquid suspensions, it being understood that suspending agents will not necessarily aid dissolution of the active ingredient in the solvent. Aqueous solvents include, for example, water and isotonic saline. Oily solvents include, for example, almond oil, oily esters, ethyl alcohol, vegetable oils such as arachis, olive, sesame, or coconut oil, fractionated vegetable oils, and mineral oils such as liquid paraffin.

Powdered and granular formulations of a pharmaceutical preparation may be prepared using known methods. Such formulations may be administered directly to a subject, used, for example, to form tablets, to fill capsules, or to prepare an aqueous or oily suspension or solution by addition of an aqueous or oily vehicle thereto. Each of these formulations may further comprise one or more of dispersing or wetting agent, a suspending agent, and a preservative. Additional excipients, such as fillers and sweetening, flavoring, or coloring agents, may also be included in these formulations.

A pharmaceutical composition may also be prepared, packaged, or sold in the form of oil-in-water emulsion or a water-in-oil emulsion. The oily phase may be a vegetable oil such as olive or arachis oil, a mineral oil such as liquid paraffin, or a combination of these. Such compositions may further comprise one or more emulsifying agents such as naturally occurring gums such as gum acacia or gum tragacanth, naturally-occurring phosphatides such as soybean or lecithin phosphatide, esters or partial esters derived from combinations of fatty acids and hexitol anhydrides such as sorbitan monooleate, and condensation products of such partial esters with ethylene oxide such as polyoxyethylene sorbitan monooleate. These emulsions may also contain additional ingredients including, for example, sweetening or flavoring agents.

A pharmaceutical composition may be prepared, packaged, or sold in a formulation suitable for rectal administration. Such a composition may be in the form of, for example, a suppository, a retention enema preparation, and a solution for rectal or colonic irrigation.

Suppository formulations may be made by combining the active ingredient with a non-irritating pharmaceutically acceptable excipient which is solid at ordinary room temperature (*i.e*., about 20°C) and which is liquid at the rectal temperature of the subject (*i.e.,* about 37°C in a healthy human). Suitable pharmaceutically acceptable excipients include, but are not limited to, cocoa butter, polyethylene glycols, and various glycerides. Suppository formulations may further comprise various additional ingredients including, but not limited to, antioxidants and preservatives.

Retention enema preparations or solutions for rectal or colonic irrigation may be made by combining the active ingredient with a pharmaceutically acceptable liquid carrier. As is well known in the art, enema preparations may be administered using, and may be packaged within, a delivery device adapted to the rectal anatomy of the subject. Enema preparations may further comprise various additional ingredients including, but not limited to, antioxidants and preservatives.

A pharmaceutical composition may be prepared, packaged, or sold in a formulation suitable for vaginal administration. Such a composition may be in the form of, for example, a suppository, an impregnated or coated vaginally-insertable material such as a tampon, a douche preparation, a gel or cream or solution for vaginal irrigation.

Methods for impregnating or coating a material with a chemical composition are known in the art, and include, but are not limited to methods of depositing or binding a chemical composition onto a surface, methods of incorporating a chemical composition into the structure of a material during the synthesis of the material (*i.e.,* such as with a physiologically degradable material), and methods of absorbing an aqueous or oily solution or suspension into an absorbent material, with or without subsequent drying.

Douche preparations or solutions for vaginal irrigation may be made by combining the active ingredient with a pharmaceutically acceptable liquid carrier. As is well known in the art, douche preparations may be administered using, and may be packaged within, a delivery device adapted to the vaginal anatomy of the subject.

Douche preparations may further comprise various additional ingredients including, but not limited to, antioxidants, antibiotics, antifungal agents, and preservatives.

Additional delivery methods for administration of compounds include a drug delivery device, such as that described in U.S. Pat. No. 5,928,195, filed on July 28, 1998.

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (*i.e.,* powder or granular) form for reconstitution with a suitable vehicle (*e.g.*, sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides. Other parentally-administrable formulations that are useful include those, which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt.

Formulations suitable for topical administration include, but are not limited to, liquid or semi-liquid preparations such as liniments, lotions, oil-in-water or water-in-oil emulsions such as creams, ointments or pastes, and solutions or suspensions. Topically-administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

A pharmaceutical composition of the invention may be prepared, packaged, or sold in a formulation suitable for pulmonary administration via the buccal cavity. Such a formulation may comprise dry particles which comprise the active ingredient and which have a diameter in the range from about 0.5 to about 7 nanometers, and preferably from about 1 to about 6 nanometers. Such compositions are conveniently in the form of dry powders for administration using a device comprising a dry powder reservoir to which a stream of propellant may be directed to disperse the powder or using a self-propelling solvent/powder-dispensing container such as a device comprising the active ingredient dissolved or suspended in a low-boiling propellant in a sealed container. Preferably, such powders comprise particles wherein at least 98% of the particles by weight have a diameter greater than 0.5 nanometers and at least 95% of the particles by number have a diameter less than 7 nanometers. More preferably, at least 95% of the particles by weight have a diameter greater than 1 nanometer and at least 90% of the particles by number have a diameter less than 6 nanometers. Dry powder compositions preferably include a solid fine powder diluent such as sugar and are conveniently provided in a unit dose form.

Low boiling propellants generally include liquid propellants having a boiling point of below 65°F at atmospheric pressure. Generally the propellant may constitute 50 to 99.9% (w/w) of the composition, and the active ingredient may constitute 0.1 to 20% (w/w) of the composition. The propellant may further comprise additional ingredients such as a liquid non-ionic or solid anionic surfactant or a solid diluent (preferably having a particle size of the same order as particles comprising the active ingredient).

Pharmaceutical compositions formulated for pulmonary delivery may also provide the active ingredient in the form of droplets of a solution or suspension. Such formulations may be prepared, packaged, or sold as aqueous or dilute alcoholic solutions or suspensions, optionally sterile, comprising the active ingredient, and may conveniently be administered using any nebulization or atomization device. Such formulations may further comprise one or more additional ingredients including, but not limited to, a flavoring agent such as saccharin sodium, a volatile oil, a buffering agent, a surface active agent, or a preservative such as methylhydroxybenzoate. The droplets provided by this route of administration preferably have an average diameter in the range from about 0.1 to about 200 nanometers.

The formulations described herein as being useful for pulmonary delivery are also useful for intranasal delivery of a pharmaceutical composition of the invention.

Another formulation suitable for intranasal administration is a coarse powder comprising the active ingredient and having an average particle from about 0.2 to 500 micrometers. Such a formulation is administered in the manner in which snuff is taken *i.e.,* by rapid inhalation through the nasal passage from a container of the powder held close to the nares.

Formulations suitable for nasal administration may, for example, comprise from about as little as 0.1% (w/w) and as much as 100% (w/w) of the active ingredient, and may further comprise one or more of the additional ingredients described herein.

A pharmaceutical composition may be prepared, packaged, or sold in a formulation suitable for buccal administration. Such formulations may, for example, be in the form of tablets or lozenges made using conventional methods, and may, for example, 0.1 to 20% (w/w) active ingredient, the balance comprising an orally dissolvable or degradable composition and, optionally, one or more of the additional ingredients described herein. Alternately, formulations suitable for buccal administration may comprise a powder or an aerosolized or atomized solution or suspension comprising the active ingredient. Such powdered, aerosolized, or aerosolized formulations, when dispersed, preferably have an average particle or droplet size in the range from about 0.1 to about 200 nanometers, and may further comprise one or more of the additional ingredients described herein.

A pharmaceutical composition may be prepared, packaged, or sold in a formulation suitable for ophthalmic administration. Such formulations may, for example, be in the form of eye drops including, for example, a 0.1-1.0% (w/w) solution or suspension of the active ingredient in an aqueous or oily liquid carrier. Such drops may further comprise buffering agents, salts, or one or more other of the additional ingredients described herein. Other ophthalmalmically-administrable formulations that are useful include those, which comprise the active ingredient in microcrystalline form or in a liposomal preparation.

The mixture of peroxiredoxin and pharmacologically acceptable additives is preferably prepared as a lyophilized product, and dissolved when in use. Such preparation can be prepared into a solution containing about 0.1-25 mg/ml of peroxiredoxin, by dissolving same in distilled water for injection or sterile purified water. More preferably, it is adjusted to have a physiologically isotonic salt concentration and a physiologically desirable pH value (pH 6-8).

While the dose is appropriately determined depending on symptom, body weight, sex, animal species and the like, it is generally assumed that treatment options holding the blood concentration at about 5 µg/ml will be preferred. This plasma concentration may be achieved through administration of one to several doses a day. When peroxiredoxin protein is to be administered to a subject, 0.1 ng to 10 mg/kg body weight (*e.g*., 1 ng to 1 mg/kg body weight) of peroxiredoxin can be given intravenously.

The compound may be administered to an animal as frequently as several times daily, or it may be administered less frequently, such as once a day, once a week, once every two weeks, once a month, or even lees frequently, such as once every several months or even once a year or less. The frequency of the dose will be readily apparent to the skilled artisan and will depend upon any number of factors, such as, but not limited to, the type and severity of the disease being treated, the type and age of the animal, etc.

The present disclosure further provides host cells genetically engineered to contain the polynucleotides. For example, such host cells may contain nucleic acids of the invention introduced into the host cell using known transformation, transfection or infection methods. The present disclosure still further provides host cells genetically engineered to express the polynucleotides of the invention, wherein such polynucleotides are in operative association with a regulatory sequence heterologous to the host cell, which drives expression of the polynucleotides in the cell.

Knowledge of nucleic acid sequences allows for modification of cells to permit, or increase, expression of endogenous polypeptide. Cells can be modified (*e.g.,* by homologous recombination) to provide increased polypeptide expression by replacing, in whole or in part, the naturally occuring promoter with all or part of a heterologous promoter so that the cells express the polypeptide at higher levels. The heterologous promoter is inserted in such a manner that it is operatively linked to the encoding sequences. See, for example, PCT International Publication No. WO 94/12650, PCT International Publication No. WO 92/20808, and PCT International Publication No. WO 91/09955. It is also contemplated that, in addition to heterologous promoter DNA, amplifiable marker DNA (*e.g.,* ada, dhfr, and the multifunctional CAD gene which encodes carbamyl phosphate synthase, aspartate transcarbamylase, and dihydroorotase) and/or intron DNA may be inserted along with the heterologous promoter DNA. If linked to the coding sequence, amplification of the marker DNA by standard selection methods results in co-amplification of the desired protein coding sequences in the cells.

The host cell can be a higher eukaryotic host cell, such as a mammalian cell, a lower eukaryotic host cell, such as a yeast cell, or the host cell can be a prokaryotic cell, such as a bacterial cell. Introduction of the recombinant construct into the host cell can be effected by calcium phosphate transfection, DEAE, dextran mediated transfection, or electroporation (Davis et al., BASIC METHODS IN MOLECULAR BIOLOGY (1986)). The host cells containing one of the polynucleotides of the invention, can be used in conventional manners to produce the gene product encoded by the isolated fragment (in the case of an ORF) or can be used to produce a heterologous protein under the control of the EMF.

Any host/vector system can be used to express one or more of the ORFs. These include, but are not limited to, eukaryotic hosts such as HeLa cells, Cv-1 cell, COS cells, 293 cells, Sf21 and Sf9 cells, as well as prokaryotic host such as *E. coli* and *B. subtilis.* The most preferred cells are those which do not normally express the particular polypeptide or protein or which expresses the polypeptide or protein at low natural level. Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., in MOLECULAR CLONING: A LABORATORY MANUAL, Second Edition, Cold Spring Harbor, New York (1989), the disclosure of which is hereby incorporated by reference.

Various mammalian cell culture systems can also be employed to express recombinant protein. Examples of mammalian expression systems include the COS-7 lines of monkey kidney fibroblasts, described by Gluzman, CELL 23: 175 (1981). Other cell lines capable of expressing a compatible vector are, for example, the C127, monkey COS cells, Chinese Hamster Ovary (CHO) cells, human kidney 293 cells, human epidermal A431 cells, human Co1o205 cells, 3T3 cells, CV-1 cells, other transformed primate cell lines, normal diploid cells, cell strains derived from *in vitro* culture of primary tissue, primary explants, HeLa cells, mouse L cells, BHK, HL-60, U937, HaK, H9 or Jurkat cells. Mammalian expression vectors will comprise an origin of replication, a suitable promoter and also any necessary ribosome binding sites, polyadenylation site, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites may be used to provide the required nontranscribed genetic elements. Recombinant polypeptides and proteins produced in bacterial culture are usually isolated by initial extraction from cell pellets, followed by one or more salting-out, aqueous ion exchange or size exclusion chromatography steps. Protein refolding steps can be used, as necessary, in completing configuration of the mature protein. Finally, high performance liquid chromatography (HPLC) can be employed for final purification steps. Microbial cells employed in expression of proteins can be disrupted by any convenient method, including freeze-thaw cycling, sonication, mechanical disruption, or use of cell lysing agents.

Alternatively, it may be possible to produce the protein in lower eukaryotes such as yeast or insects or in prokaryotes such as bacteria. Potentially suitable yeast strains include *Saccharomyces cerevisiae, Schizosaccharomyces pombe, Kluyveromyces* strains, *Candida,* or any yeast strain capable of expressing heterologous proteins. Potentially suitable bacterial strains include *Escherichia coli, Bacillus subtilis, Salmonella typhimurium,* or any bacterial strain capable of expressing heterologous proteins. If the protein is made in yeast or bacteria, it may be necessary to modify the protein produced therein, for example by phosphorylation or glycosylation of the appropriate sites, in order to obtain the functional protein. Such covalent attachments may be accomplished using known chemical or enzymatic methods.

In another embodiment, cells and tissues maybe engineered to express an endogenous gene comprising the polynucleotides of the invention under the control of inducible regulatory elements, in which case the regulatory sequences of the endogenous gene may be replaced by homologous recombination. As described herein, gene targeting can be used to replace a gene's existing regulatory region with a regulatory sequence isolated from a different gene or a novel regulatory sequence synthesized by genetic engineering methods. Such regulatory sequences may be comprised of promoters, enhancers, scaffold-attachment regions, negative regulatory elements, transcriptional initiation sites, regulatory protein binding sites or combinations of said sequences. Alternatively, sequences, which affect the structure or stability of the RNA or protein produced, may be replaced, removed, added, or otherwise modified by targeting. These sequence include polyadenylation signals, mRNA stability elements, splice sites, leader sequences for enhancing or modifying transport or secretion properties of the protein, or other sequences that alter or improve the function or stability of protein or RNA molecules.

The targeting event may be a simple insertion of the regulatory sequence, placing the gene under the control of the new regulatory sequence, e.g., inserting a new promoter or enhancer or both upstream of a gene. Alternatively, the targeting event may be a simple deletion of a regulatory element, such as the deletion of a tissue-specific negative regulatory element. Alternatively, the targeting event may replace an existing element; for example, a tissue-specific enhancer can be replaced by an enhancer that has broader or different cell-type specificity than the naturally occurring elements. Here, the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the host cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

The gene targeting or gene activation techniques, which can be used in accordance with this aspect of the invention, are more particularly described in U.S. Patent No. 5,272,071 to Chappel; U.S. Patent No. 5,578,461 to Sherwin et al.,; International Application No. PCT/US92/09627 (WO 93/09222) by Selden et al.,; and International Application No. PCT/US90/06436 (WO 91/06667) by Skoultchi et al*..*

### EXAMPLES

These Examples are provided for the purpose of illustration only and the invention should in no way be construed as being limited to these Examples, but rather should be construed to encompass any and all variations which become evident as a result of the teaching provided herein.

### EXAMPLE 1. DETERMINATION OF THE ANTIVIRAL ACTIVITY OF TWO PEROXIREDOXINS (NKEF-A AND NKEF-B) PRODUCED BY CD8+ T-CELLS OF HIV-1 INFECTED PERSONS

### Differential gene expression in CD8⁺ T-cells of HIV-1 seronegative and seropositive individuals

CD8⁺ T-cells are a major source for inhibitory non-cytoloytic factors in HIV-1 infected persons. It has been shown that secretion of soluble antiviral factors is elevated in expanded CD8⁺ T-cells from HIV infected persons (Sauri et al., J LBUKOC BIOL 59: 925-931 (1996)). Potential differences in mRNA gene expression were evaluated using the ATLAS array for 588 different genes. Expanded CD8⁺ T-cell populations of HIV-1 seronegative and the HIV-1 seropositive untreated individuals were evaluated prior to stimulation and 4 h following stimulation with anti-CD3 antibodies.

Although the ATLAS array includes a wide spectrum of gene populations significant differences were limited to the expression of only 4 genes. These included significant differences (*p*<0.001) in mRNA expression for chemokine I-309, the cytokines GM-CSF and IL-13, and the peroxiredoxin gene NKEF-B (FIG. 1A). The peroxiredoxin data were confirmed by Northern-blot analysis using NKEF-A, a homologue of NKEF-B showing significant differences for this peroxiredoxin gene (FIG 1B). These mRNA results are consistent with previous studies showing higher secretion of I-309, GM-CSF and IL-13 in stimulated CD8⁺ T-cells from seropositive persons (Geiben-Lynn et al., J VIROL 75: 8306-16 (2001)), and they extend these prior studies by demonstrating the elevation in a peroxiredoxin family mRNA in CD8⁺ T-cells from HIV-1 seropositive persons.

Plasma was obtained from 13 long-term nonprogressors. All had been infected for more than 10 years, had plasma HIV-1 loads <400 RNA copies per ml and CD4⁺ T-cell counts >500 per µl in the absence of therapy. Control plasma samples were obtained from 13 HIV-1 seronegative, healthy donors. Additional plasma samples from 6 asymptomatic, 5 symptomatic and 2 AIDS patients, all under HAART treatment, were investigated.

Bulk CD8⁺ T-cells were purified, propagated and simulated as previously described (Geiben-Lynn et al., J VIROL 75: 8306-16, 2001) by anti-CD3 crosslinking from peripheral blood mononuclear cells (PBMCs), which were obtained from six HIV-1 infected long-term non-progessors (17393,15188, CTS-02, NEW, RK2000, CX741) (Geiben-Lynn et al., J VIROL 75: 8306-16 (2001)) and from seven HIV-1 seronegative individuals.

For total RNA extraction cell pellets of 0 h or 4 h anti-CD3-activated bulk CD8⁺ T-cells (10⁷ cells per reaction) were lysed with 1 ml of RNA STAT60 (Tel-Test B, Friendwood, TX) and the cellular RNA was purified using the RNA STAT60 protocol. To eliminate the DNA contaminant of the RNA the CleanMessage™ Kit (Genhunter, Nashville, TN) was used. Total cellular RNA (10 µg) was fractionated on a 1.2% agarose/0.7% formaldehyde gel and transferred to a GeneScreen membrane (Dupont, Wilmington, DE). Glyceraldehyde-3-phosphate dehydrogenase (GADPH) specific DNA probes (Clontech) and NKEF-A specific DNA probes (Shau et al., IMMUNOGENETICS 40: 129-34, 1994) were ³²P-radiolabelled using the DECAprime™ Kit (Ambion, Austin, TX). Membranes were sequentially hybridized with the ³²P-radiolabelled cDNA probes. Blots were washed at high stringency (0.2 x SSC, 55°C) and exposed on a Kodak X-OMAT Autoradiography Film (Kodak, Rochester, NY) or measured with Molecular Phosphorimager System GS-363 (Bio-Rad, Hercules, CA) for an equal amount of time. Signal intensity calculations were performed using the supporting software program Molecular Analyst^{™} and calculated against the GAPDH signal.

The Atlas Array™ (Clontech) is a nitrocellulose membrane with 588 spotted cDNAs which include oncogenes, tumorsuppressor genes, cell-cycle regulators genes, stress response genes, ion channels and ion transport genes, intracellular signal transduction modulator genes, intracellular signal transduction effectors genes, apoptosis-related genes, DNA repair and recombination genes, transcription factor genes, general DNA-binding protein genes, receptors of growth factor genes, cell-surface antigens genes, cell adhesion genes and cell-cell communication genes (Clontech manufacturer's guide).

For the hybridization polyadenylated (Poly A⁺) mRNA was prepared from 100 µg total RNA of 4 h CD3-crosslinked or untreated bulk CD8⁺ T-cells. The mRNA was suspended in DEPC-treated water and separated on Poly(A) Quik® mRNA Columns (Stratagene, La Jolla, CA) according to the manufacturer's protocol. Poly A⁺ mRNA (1 µg in 2 µl) of each sample was transcribed to radiolabeled cDNA using 1 µl MMLV Reverse Transcriptase (50 U/µl; Stratagene) and 3.5 µl [α-³²P]-dATP (3000 Ci/mmol, 10 mCi/ml) according to the ATLAS assay protocol and used for hybridization. The binding of radioactivity to membrane was measured with the Molecular Phosphorimager System GS-363 (Bio-Rad) for an equal amount of time. Signal intensity calculations were performed using the supporting software program Molecular Analyst^{™} and calculated against the GAPDH signal.

### Inhibition of HIV-1 replication with recombinant NKEF-A and NKEF-B protein

Having demonstrated that GM-CSF, I-309, IL-13 and the NKEFs are more highly expressed in HIV-1 infected untreated individuals, it was next evaluated whether some of these gene products might contribute to the previously observed antiviral activity of CD8⁺ T-cells (Geiben-Lynn et al., J VIROL 75: 8306-16, 2001). GM-CSF, I-309 and IL-13 have been shown to influence HIV-1 replication in some *in vitro* systems (Crowe et al., J LEUKOC BIOL 62: 41-8, 1997). X4 HTLV-IIIB (ATCC No. CRL-8543, hereinafter HIV-1_{IIIB}; Chang et al., NATURE, 363: 466-9 (1993)), a prototypical T-tropic strain of HIV (American Type Tissue Collection, Monassass, VA, USA; ATCC No. CRL-8543), was used to assess the effect of peroxiredoxins on T-tropic HIV infection. Human T lymphoblastoid cells (H9 cells) expressing the human leukocyte antigen proteins (HLA) B6, Bw62, and Cw3 were acutely infected with X4 HIV at a MOI of 1 x 10⁻² TCID₅₀ per milliliter. The quantity of virus in a specified suspension volume (*e.g.,* 0.1 ml) that will infect 50% of a number (n) of cell culture microplate wells, or tubes, is termed the Tissue Culture Infectious Dose 50 [TCID₅₀]. TCID₅₀ is used as an alternative to determining virus titre by plaqueing (which gives values as PFUs or plaque-forming units). Karber, 1931. H9 cells (HLAA1, B6, Bw62, Cw3) were acutely infected with HIV-1_{IIIB} at a MOI of 10⁻² TCID₅₀ per milliliter and resuspended in RPMI 1640 (Sigma, St Louis, Mo.) supplemented with 20% (v/v) heat-inactivated fetal calf serum (Sigma; R20 medium) (Geiben-Lynn et al., J VIROL 75: 8306-16, 2001). The infected H9 cells were resuspended to 5 x 10⁵ cells/ml in R20 cell culture medium. Two milliliters of this suspension was pipetted into each well of a 24-well microtiter plate. H9 cell supernatant (1 ml) was removed every 3 days and replaced with 1 ml fresh R20 medium supplemented with recombinant NKEF-A or NKEF-B protein. After 9 days the concentration of p24 antigen was measured using an HIV-1 p24 ELISA kit (NEN Life Science, Boston, MA).

Simian immunodeficiency virus (SIV) belongs to the family *Retroviridae* (subfamily *Lentivirinae*) and is closely related to human immunodeficiency virus types 1 and 2 (HIV-1 and HIV-2), the etiologic agents of AIDS. Originally reported in 1985, the first isolate from a rhesus macaque was called simian T-lymphotropic virus III (STLV-III). The SIVmac239 viral strain (hereinafter SIV₂₃₉; P. Johnson, Harvard Medical School, Boston, MA, USA) used in these studies is a dnal-tropic infectious virus that induces AIDS in rhesus macaque monkeys.

SHIV_{KU-1} (Narayan and Joag, AIDS Research and Reference Program, Division of AIDS, NIADS, Bethesda, MD, USA) is a second dual-tropic strain of SIV used in these studies. SHIV_{KU-1} is a biologically-pure suspension of SHIV that is highly pathogenic in pigtailed macaques. The virus was derived by sequential passage of the molecular construct of SIV(mac)239XHIV-1-HxB2 through bone marrow of pigtailed macaque monkeys (Joag et al., J. VIROLOGY, 70: 3189-3197 (1996)).

The cell tropism of SIV in culture depends partially on the strain of virus propagated and conditions of cell culture. In the present studies, macaque T-cell line SEM-174 cells were acutely infected with either SIV₂₃₉ or SHIV_{KU-1} at a MOI of 1 x 10⁻² TCID₅₀ per milliliter. After 9 days the concentration of p27 antigen was measured by a p27 ELISA (Coulter, Miami, FL).

The medium controls for the HIV or SIV experiments demonstrated p24- or p27 antigen levels in excess of 100 ng/ml at day 9, and were used to calculate percent virus inhibition.

None of the chemokines or cytokines (GM-CSF, I-309, IL-13) found being more highly expressed in HIV-1 seropositive untreated individuals inhibited HIV-1_{IIIB} replication of acutely infected CD4⁺ T-cells in the system used when added up to 1 µg/ml, demonstrating that these proteins do not contribute to the observed inhibition (Geiben-Lynn et al., J VIROL 75: 8306-16, 2001). In contrast it was found that NKEF-A and NKEF-B purified recombinant proteins (FIG. 4A) inhibited HIV-1_{IIIB} at an ID₅₀ of 130 nM (3 µg/ml), respectively, similar to Stromal-Derived Factor (SDF-1), the only natural occurring CXCR4 ligand found so far ((Geiben-Lynn et al., J VIROL 75: 8306-16, 2001). Additionally, using the NKEF-B protein for the inhibition assay with SHIV and SIV strains, it was found that nearly complete suppression of these viruses at 3 µg/ml (FIG. 4 C/D). The observed inhibition did not correlate with a decrease in cell count as measured at log phase of cell growth from day 2-6 by tryphan blue staining (data not shown). Our data indicate that recombinant NKEFs can inhibit replication of T-tropic HIV-1, SIV and SHIV.

### Secretion of NKEF-A and NKEF-B proteins by CD8⁺ T-cells

Having demonstrated that the NKEFs can inhibit HIV-1 replication, it was next tested whether the NKEFs are secreted, and whether these proteins contribute to the antiviral activity of CD8⁺ T-cells (Geiben-Lynn et al., J VIROL 75: 8306-16,2001). Although NKEF-A and NKEF-B proteins were originally described as endogenous proteins (Shau et al., BIOCHEM BIOPHYS RES COMM 199: 83-8, 1994), another protein of the peroxiredoxin family, thioredoxin, also termed adult T-cell leukemia (ATL)-derived factor (ADF), has been shown to be secreted through a novel pathway despite having no secretion leader sequence which was confirmed by another member of the peroxiredoxin family (Rubartelli et al., J BIOL CHEM 267: 24161-4, 1992; Okado-Matsumoto et al., J BIOCHEM (TOKYO) 127: 493-501, 2000). It was found that both NKEF-A and NKEF-B proteins were secreted after 4 h, regardless of whether the cells were stimulated. The concentrations produced averaged 15 - 40 ng/ml (FIG 2), or at least 10 - 20 times higher than seen for the chemokines and cytokines at this 4 h timepoint (Geiben-Lynn et al., J VIROL 75: 8306-16, 2001). The secretion was observed in stimulated CD8⁺ T-cell from both infected or uninfected individuals (FIG 2). The finding of increased secretion in the supernatants of activated CD8⁺ T-cells from both of infected and uninfected individuals indicates that although NKEFs are able to exert antiviral activity they are not the elusive CAF. Additionally, the concentrations of secreted NKEFs at 4 h (15 - 40 ng/ml) are at levels below those causing significant inhibition.

### Blood plasma levels of the NKEF-A and NKEF-B proteins

Having demonstrated that the NKEFs are secreted, blood plasma levels were tested to evaluate if these concentrations were sufficient to mediate inhibition *in vivo.* Additionally, blood plasma levels were examined to determine if they are dependent on whether individuals are infected or not and treated or untreated. Therefor blood plasma levels for NKEF-A and NKEF-B were evaluated using a specific ELISA. No significant difference was found in NKEF-A or NKEF-B plasma levels between these populations. Nevertheless plasma levels of the NKEFs were elevated up to 500 ng/ml in 3 individuals of 13 (∼23%) untreated non-progressing patients tested with levels 2.5-8 times higher than the uninfected or treated HIV patients (FIG. 3). NKEF-A and NKEF-B concentrations were comparable in the patients tested, and were found at high levels up to 1 µg/ml NKEFs when the NKEF-A and NKEF-B concentrations were added. At this concentration, HIV-1 inhibition was detectable *in vitro* (FIG. 2B), and an increase of NK cell activity *in vitro* has been noted (Sauri et al., J LEUKOC BIOL 59: 925-31 (1996)), demonstrating that NKEFs might have an antiviral influence *in vivo.* Our data indicate that elevated levels of NKEFs might be typical for a small percentage of untreated HIV-1 infected individuals.

For the NKEF-A and NKEF-B ELISAs, 2 µg/ml of monoclonal mouse anti-NKEF-A or anti-NKEF-B antibody (Pharmigen) was incubated overnight at 4°C on protein high-binding E.I.A./R.I.A. plates (Costar, Cambridge, MA) in coating buffer (0.05 M CO3-2/HCO3- buffer, pH 9.6). Plates were washed with PBST buffer (phospate-buffered saline (PBS), 0.05% (v/v) Triton X100 (Sigma), pH 7.4) and blocked for 2 h at 37°C with blocking buffer (PBS, 3% (v/v) goat serum, 3% (w/v) bovine serum albumin (BSA). A rabbit polyclonal NKEF-A/NKEF-B detection antibody (Shau et al., CELL IMMUNOL 147: 1-11 (1993)) was used 1:1000 incubated in PBSBT buffer (PBS/0.1% (w/v) BSA/0.05% (v/v) Triton X100) at 37°C and 30 min. After washing with PBST buffer, a horseradish peroxidase-coupled anti-rabbit antibody (Vector, Burlingame, CA) was used at 1: 50,000 dilution at room temperature for 20 min. After washing with PBST buffer the ELISA was developed for 30 min at room temperature with a 1:1 dilution of peroxidase solution B and TMB peroxidase substrate (Kirkeguard & Perry Laboratories, Gaithersburg, Maryland) and stopped with 4 N H2SO4. SDS-PAGE and Western-blot were carried out as described previously using polyclonal NKEF-A and NKEF-B specific antibodies (Shau et al., CELL IMMUNOL 147: 1-11 (1993)) at a dilution of 1: 10,000. Protein concentration was determined by the BCA method (Pierce, Rockford, IL).

### Inhibition of HIV-1 replication through NKEF-A and NKEF-B transfection

Having demonstrated that a small percentage of HIV untreated individuals have a higher level of NKEFs in plasma, the ability of T-cells overexpressing the NKEFs to inhibit HIV replication was examined since it was already shown that a higher gene expression of NKEFs was correlated with Tₕ1-responding CD4⁺T-cells and that Tₕ1 response was related to a better outcome in the HIV-1 disease (Nagai et al., INT IMMUNOL 13: 367-376 (2001); Barker et al., PROC NATAL ACAD SCI USA 92: 11135-11139 (1995)). Furthermore, it was shown for antioxidant enzyme (AOE372) that transfected T-cells could block HIV-1 replication (Jin et al., J BIOL CHBM 272: 30952-30961 (1997)). Transfected Jurkat T-cell overexpressed intracellular NKEF levels approximately 10 times (FIG. 5A). These NKEF-A and NKEF-B gene transfected T-cells blocked 80-98% HIV-1_{IIIB} replication starting at day 6 post infection (FIG 5B). Our data indicate that enhanced expression of NKEFs in T-cells might inhibit HIV-1 replication, providing further evidence of antiviral effect of these compounds.

For the inhibition experiments with NKEF-A and NKEF-B transfected cells, Jurkat - CD4⁺ T-cells and H9 CD4⁺ T-cells were used for cloning. NKEF-A- and NKEF-B-pBacPAK9 vectors (Sauri et al., BIOCBEM BIOPHYS RES COMM 208: 964-9, 1995) were digested with BamH1 and Xho1 (New England Biolabs, Beverly, MA). The digest was treated with T4 polymerase (Gibco, Grand Island, NY) for blunt-end ligation according to the manufacturer's instructions. The NKEF-A and NKEF-B fragments were then inserted into the SmaI cloning side of the pIRES2-EGFP expression vector (Clontech, Palo Alto, CA) and cultured in *E. coli.* Plasmid DNAs were purified and the direction of the inserts was confirmed by DNA sequencing. Jurkat cells were then transfected with a DNA-liposome mixture (Fugene, Roche Diagnostics, Indianapolis, IN) and selected under G418 (Sigma, St. Louis, Mo.) pressure (1.5 mg/ml). Stably transfected cells were then used in the above described inhibition test. For day 1-9, the concentration of p24 antigen was measured using an HIV-1 p24 ELISA kit (NEN, Life Science, Boston, MA). The percentage of inhibition was calculated against mock-transfected cells.

To produce the NKEF-A and NKEF-B proteins for the above described inhibition test, the NKEF-A and NKEF-B genes were cloned into the baculovirus expression vector pBacPAK9 (Clontech) and overexpressed in *Spodoptera frugiperda* (Sf21 cells; Clontech) as described (Sauri et al., BIOCHBM BIOPHYS RES COMM 208: 964-9, 1995). After transfections, Sf21 cells were harvested and lysed with insect lysis buffer (Pharmigen, San Diego, CA) at day 2-4. Afterwards recombinant protein was purified as described earlier (Sauri et al., J LEUKOC BIOL 59: 925-31 (1995).

Fisher's exact test was used to determine significance. Standard error is shown as error bars.

### In vivo evaluation of peroxiredoxin antiretroviral activity

There is currently no standard *in vivo* animal model endorsed by the U.S. Food and Drug Administration for the evaluation of antiretroviral agents such as the peroxiredoxin, nor is an *in vivo* model necessary for IND approval in the US. Human cell lines can, however, be cultivated in hollow fibers in the subcutaneous and intraperitoneal compartments of mice (Hollingshead et al., LIFE SCI., 57: 131-41 (1995)). *In vivo* evaluation of peroxiredoxin antiretroviral activity can be evaluated in the murine hollow fiber model developed by Hollingshead and coworkers (ANTIVIRAL RES., 28: 256-79 (1995)).

H9 or PM1 cell-bearing polyvinylidene fluoride fibers (500,000 Mw cutoff; 1 mm LD.; Spectrum Medical Corp., Houston, TX, USA) are prepared by filling conditioned hollow fibers with cell inoculum (uninfected cells, acutely HIV infected cells or chronically HIV infected cells) (Hollingshead et al., LIFE SCI., 57: 131-41 (1995)). These inoculated hollow fibers are surgically implanted either subcutaneously or in the peritoneal cavity of SCID mice (SCID/NCr; NCI Animal Production Facility, NCI-FCRDC, Frederick, MD, USA). Hollow-fiber-bearing SCID mice are dosed either acutely or chronically with increasing amounts of purified peroxiredoxin preparation. The peroxiredoxin preparation (3-500 µg per mouse/day) are administered to the hollow-fiber-bearing SCID mice by subcutaneous injection, intraperitoneal injection, intravenous or oral routes. At select times, blood is sampled from control and test animals and serum prepared. The amount of viral particles in test and control serum is measured by p24 ELISA. Peroxiredoxin antiviral action yield a significant decrease in viral load, as judged by at least a 15% decrease in serum p24 protein content in peroxiredoxin-treated animals relative to the serum p24 content of the untreated control animals.

### EQUIVALENTS

Although particular embodiments have been disclosed herein in detail, this has been done by way of example for purposes of illustration only. In particular it is contemplated by the inventors that various substitutions, alterations, and modifications may be made to the invention without departing from the scope of the invention as defined by the claims. Other aspects, advantages, and modifications are within the scope of the invention. For example, peroxiredoxin can be used as an antiviral drug against other viruses (*e.g*., HTL-1, HTL-2, HSV, EBV, HBV, HCV, or CMV).

## Claims

1. Use for the preparation of a medicament for the prevention or treatment of viral infection, of a sustantially pure peroxiredoxin selected from
(i) NKEF-A,
(ii) NKEF-B or
(iii) a peroxiredoxin having at least 90% identity with NKEF-A or NKEF-B and yielding a significant decrease in viral load, as judged by at least a 15% decrease in serum p24 protein content in peroxiredoxin-treated animals relative to the serum p24 content of untreated control animals.

2. The use of claim 1, wherein the peroxiredoxin is protease-resistant.

## Patentansprüche

1. Verwendung, zur Herstellung eines Medikaments zur Vorbeugung oder Behandlung einer viralen Infektion, eines im wesentlichen reinen Peroxiredoxins, ausgewählt aus
(i) NKEF-A,
(ii) NKEF-B oder
(iii) eines Peroxiredoxins, das mindestens 90 % Identität mit NKEF-A oder NKEF-B aufweist und zu einer signifikanten Abnahme der Virusbelastung führt, beurteilt aufgrund einer mindestens 15 %igen Abnahme des p24-Serumproteingehalts in Peroxiredoxin-behandelten Tieren relativ zu dem p24-Serumgehalt von unbehandelten Kontrolltieren.

2. Verwendung nach Anspruch 1, wobei das Peroxiredoxin proteaseresistent ist.

## Revendications

1. Utilisation pour l'obtention d'un médicament destiné à la prévention ou au traitement d'une infection virale, d'une peroxyrédoxine sensiblement pure choisie entre
(i) NKEF-A,
(ii) NKEF-B ou
(iii) une peroxyrédoxine ayant au moins 90% d'identité avec NKEF-A ou NKEF-B et donnant une diminution signifiante de la chargé virale comme en tant que jugée par une diminution d'au moins 15 % de la teneur du protéine p24 dans le sérum des animaux traités avec la peroxyrédoxine par rapport à la teneur du protéine p24 dans le sérum des animaux de référence non traités.

2. Utilisation selon la revendication 1, dans laquelle la peroxyrédoxine est résistante aux protéases.
